# EUROPÄISCHE PATENTSCHRIFT

(11) **EP 2 050 435 B1**
(45) Veröffentlichungstag und Bekanntmachung des Hinweises auf die Patenterteilung: **25.05.2011**
(21) Anmeldenummer: 08167006.9
(22) Anmeldetag: 20.10.2008
(51) Int. Cl.: A61P 11/14, A61K 9/08, A61K 9/28, A61K 31/00, A61K 9/00, A61K 45/06, A61K 31/485, A61K 47/46, A61K 47/10

(54) **Hustenpräparat**
Cough mixture
Préparation pour la toux

(30) Priorität: 18.10.2007 DE 102007000521
(43) Veröffentlichungstag der Anmeldung: 22.04.2009
(73) Patentinhaber: Conrad, Renate, 40789 Monheim (DE)
(72) Erfinder: Conrad, Renate, 40789 Monheim (DE)
(74) Vertreter: Gille Hrabal Struck Neidlein Prop Roos

(56) Entgegenhaltungen:
- EP-A- 0 679 391
- EP-A- 1 891 936
- WO-A-00/41693
- WO-A-2007/054958
- WO-A-2007/084331
- US-A- 5 141 961
- US-A- 5 510 389
- US-A1- 2003 118 613
- HOBBY ET AL: "Cough, its pathology and management" AMERICAN JOURNAL OF SURGERY, PAUL HOEBER, NEW YORK, NY, US, Bd. 89, Nr. 2, 1. Februar 1955 (1955-02-01), Seiten 285-293, XP023224614 ISSN: 0002-9610 [gefunden am 1955-02-01]

## Beschreibung

Die vorliegende Erfindung betrifft ein Hustenpräparat, genauer ein Hustenpräparat, welches Dextromethorphan oder ein pharmazeutisch verträgliches Salz davon, und Liquor Ammonii anisatus, als Wirkstoffe umfasst.

### Stand der Technik

Husten ist das willkürlich oder aufgrund eines Hustenreizes auftretende unwillkürliche, explosionsartige Ausstoßen von Luft. Husten ist ein Symptom und keine eigenständige Krankheit. Die Ursachen können in Erkrankungen der Atmungsorgane wie Bronchitis, Erkältung, Asthma bronchiale oder chronischobstruktiver Lungenerkrankung, des Herzens, des Magens, der Einnahme von Medikamenten (wie z.B. ACE-Hemmern) oder selten psychischen Störungen bestehen. Ein trockener Husten ohne Auswurf von Bronchialschleim, der in der Frühphase einer Erkältung, durch Allergien oder aufgrund von Arzneimitteleinnahme 8z.B. ACE-Hemmer) auftreten kann, wird auch als Reizhusten bezeichnet, Im Verlauf einer Erkältung ist der Husten zunächst trocken und wandelt sich dann nach 1 bis 3 Tagen in verschleimten Husten, bei dem es zu Absonderung von Schleim kommt, der klar oder gelbgrün gefärbt sein kann. Grundsätzlich ist die zugrundeliegende Erkrankung zu behandeln. Da der Husten aber oft als quälend empfunden wird und häufig die Nachtruhe verhindert und so die Gesundung und Erholung des Patienten verzögert, besteht ein großer Bedarf an Arzneimitteln zur Behandlung des Reizhustens und des verschleimten Hustens. Dabei ist zu beachten, dass der produktive (verschleimte) Husten ein Schutzreflex ist, der eine Verlegung der Atemwege verhindern soll, und daher nur bei starker Beeinträchtigung der Nachtruhe unterdrückt werden sollte.

Grundsätzlich stehen mehrer Typen von Therapeutika zur Behandlung des Hustens zur Verfügung.

Die Antifussiva oder auch Hustenstiller sind Mittel, die den Hustenreiz unterdrücken. Diese Substanzen wirken über das Zentralnervensystem und haben einen dämpfenden Effekt auf das Hustenzentrum im Stammhirn sowie einen sedierenden Effekt. Es handelt sich überwiegend um Opiatabkömmlinge. Hauptsächlich angewendet werden Codein, Dihydrocodein oder Hydrocodon, die alle verschreibungspflichtig sind, z.T. dem Betäubungsmittelgesetz unterliegen, ein relativ hohes Suchtpotential aufweisen und die Gefahr des Missbrauchs durch Drogenabhängige bieten.

Daneben ist auch Dextromethorphan oder dessen Salze sehr gebräuchlich. Dieser Wirkstoff ist nicht verschreibungspflichtig und zeigt ein wesentlich geringeres Suchtpotential als die übrigen vorstehend genannten Opiatderivate, Er ist in vielen kommerziell verfügbaren Hustenpräparaten enthalten, z.B. Wick® Formel 44 Hustenstiller, Wick® MediNait, Ratiopharm® Hustenstiller-Kapseln, Contac® Erkältungstrunk Forte, Silomat® DMP,

Neuere Antitussiva sind z.B. Clobutinol oder Pentoxyverin, Clobutinol ist ein Wirkstoff zur Behandlung eines trockenen Reizhustens und wird dafür bereits seit mehr als 40 Jahren eingesetzt. Er unterliegt nicht der Verschreibungspflicht. Seit dem 31. August 2007 dürfen Clobutinol-haltigen Arzneimittel nicht mehr in den Verkehr gebracht werden. Das hat das Bundesinstitut für Arzneimittel und Medizinprodukte (BfArM) angeordnet. Es forderte Patienten auf, Clobutinol-haltige Arzneimittel nicht länger einzunehmen. Der Grund für diese Sicherheitsentscheidung ist, dass dem BfArM vom Zulassungsinhaber des Originalarzneimittels neue Informationen und Daten vorgelegt wurden, die vermuten lassen, das Clobutinol in seltenen Fällen Herzrhythmusstörungen auslöst (Torsades de pointes). Das BfArM hatte zu diesem Risikoverdacht entsprechende experimentelle und klinische Untersuchungen angefordert. Es bewertet, ebenso wie der Originalhersteller, das Nutzen-Schaden-Verhältnis von Clobutinol auf der Grundlage der neuesten Erkenntnisse so, dass ein weiterer Vertrieb und die Anwendung dieser Arzneimittel medizinisch nicht vertretbar sind.

Damit steht den durch Reizhusten betroffenen Patienten ein seit fast 50 Jahren erfolgreich eingesetzter Wirkstoff nicht mehr zur Verfügung, so dass aktuell ein dringender Bedarf an gut verträglichen, wirksamen Hustenpräparaten besteht.

Als weitere Wirkstoffiklasse werden zur Erleichterung des Abhustens von Bronchialschleim sogenannte Expektorantien eingesetzt. Hier unterscheidet man zwischen Sekretolytika, die eine Verflüssigung des Bronchialsekrets bewirken, und Sekretomotorika, durch die ein verstärkter Abtransport des Schleims ausgelöst wird.

Als Sekretolytika gelten z.B. Acetylcystein, Carbocystein, Bromhexin und dessen Metabolit Ambroxol, Ammoniumchlorid und Guaifiensin, aber auch pflanzliche Produkte wie Fenchelöl oder Anisöl.

Eine gleichzeitige Einnahme von Expektorantien (Schleimlösern) und Antitussiva (Hustenstillern) ist kontraproduktiv, da das Abhusten des Bronchialschleims auf einen ungestörten Hustenreflex angewiesen ist. Wenn der entstandene Schleim nicht abgehustet werden kann, kann es zu Komplikationen wie Lungenentzündung kommen, die vermieden werden müssen. Üblicherweise gibt man Expektorantien daher tagsüber bis spätestens 1 7 Uhr, und nachts dann zur Sicherung der Nachtruhe Hustenstiller, d.h. beide Medikamententypen werden allenfalls abwechselnd, aber niemals gleichzeitig verabreicht.

Dennoch werden in der Patentliteratur einige Kombinationspräparate, die den Wirkstoff Dextrometorphan enthalten, beschrieben.

WO 92/07559 offenbart eine Kombination von Dextromethorphan mit Guiafensin, einem Expectorans, die eine pH von 8 bis 1 1 aufweisen muss.

WO 85/04589 beschreibt eine Kombination, umfassend einen nichtsteroidales antientzündliches Wirkstoff mit mindestens einer Komponente, die ausgewählt wird aus Antihistaminen, abschwellenden Mitteln, Antitussiva oder Expectorantien.

WO 99/32119 offenbart eine analgetisch wirksame Kombination aus einem Opioid-Agonisten und einem Opioid-Antagonisten ggf. in Kombination mit einem Expectorans. Durch diese Kombination soll das Suchtpotential des Opioid-Agonisten vermindert werden.

WO 03/061642 beschreibt eine Kombination eines Opiods mit einem Antitussivum, nämlich Ambroxol.

Die US Offenlegung US5141961 beschreibt eine Mischung von Dextromethorphan und Guaifensesin in ethanolischer Lösung, die gerührt wird, bis eine homogene Lösung entstanden ist.

Die internationale Anmeldung W02007084331 offenbart Dextromethorphan in einer Wirkstoffdreierkombination und Ammoniumchlorid oder Ammoniumcarbonat und weiterhin Anisöl als Geschmacksstoff aber keinen Alkohol. In der bevorzugten Ausführungsform ist sogar Ethanol ausdrücklich ausgeschlossen.

Die US2003118613 beschäftigt sich mit der verbesserten Wirksamkeit von Husten lindernden Medikamenten. Die Wirkstoffe oder deren Kombinationen können aus verschiedenen Gruppen ausgewählt werden wie z.B. der Antitussiva sowie der Expectorants. Es werden Dextromethorphan und Ammoniumchlorid nicht ausdrücklich als Kombination erwähnt geschweige denn Liquor Ammonii anisatus.

US5510389 befasst sich mit der verbesserten Stabilität von Acetaminophen, das zwar mit Dextromethorphan kombiniert werden kann, wenn es aus einer Liste von Wirkstoffen ausgewählt wird. Allerdings fehlt Liquor Ammonii anisatus.

EP0679391 beschreibt Formulierungen einer festen, schluckbaren, Husten lindernden Tablette, die ein Analgetikum und ein Antitussivum sowie Menthol enthält, aber nicht die explizite Kombination aus Dextromethorphan mit Liquor Ammonii anisatus.

Es sind aber keine Kombinationen eines Antutussivums mit einem Expectorans in alkoholischer Lösung bekannt. Auch Kombinationen von Dextromethorphan oder pharmazeutische verträglichen Salzen davon mit Anisöl bzw. Liquor Ammonii anisatus sind im Stand der Technik nicht bekannt.

Besonders für Hustenpatienten, die an Asthma bronchiale leiden, existierte aber bisher kein geeignetes Mittel zur Behandlung des Reizhustens, da sich bei Einnahme der üblichen Antitussiva üblicherweise bei dieser Patientengruppe eine extrem stake Verschleimung der Bronchien einstellt.

Die Aufgabe der Erfindung liegt daher in der Bereitstellung eines Hustenpräparats, das insbesondere auch für die Behandlung des Reizhustens bei Asthmatikern geeignet ist.

### Offenbarung der Erfindung

Es wurde nun gefunden, dass eine Kombination von Dextromethorphan oder einem pharmazeutisch vertraglichem Salz davon mit Liquor Ammonii anisatus überraschenderweise eine besonders gute Wirkung als Hustenmittel zeigt, vor allem bei Asthmatikern, die an Reizhusten leiden, da diese Kombination unerwartet gerade bei dieser Patientengruppe keine stärkere Verschleimung bewirkt und gleichzeitig zu einer deutlichen Linderung des Hustenreizes führt. Dies ist insbesondere überraschend, weil die Kombination von Expectorantien mit Antutussiva im Allgemeinen, wie vorstehend erwähnt, im Stand der Technik, grundsätzlich als ungeeignet und kontraproduktiv zur Behandlung von Husten angesehen wird.

Erfindungsgemäß wird daher bevorzugt eine Kombination von Dextromethorphan oder einem pharmazeutisch verträglichen Salz davon mit Liquor Ammonii anisatus eingesetzt.

Dextromethorphan ist ein bekanntes Antitussivum. Die chemische Bezeichnung lautet (+)-3-Methoxy-N-methylmorphinan. Es handelt sich um das (+)-Enantiomer von Racemetorphan. Die Herstellung ist beispielsweise in US 2,676,1 77 beschrieben. Der Wirkstoff ist kommerziell erhältlich. Dextromethorphan ist in zahlreichen Husten- und Erkältungsmitteln enthalten, die in rezeptfrei erhältlich sind. Es besteht die Gefahr des Missbrauchs; der Wirkstoff ist unter Drogenkonsumenten auch unter den Bezeichnungen "DXM", "DM", "CCC", "Triple C", "Candy", "Robo", "Velvet" oder "Rojo"bekannt. Während der Wirkstoff in der vorgesehenen Dosierung in Hustenmittel gut verträglich ist, kann es bei einer akuten Überdosierung zu schweren Nebenwirkungen kommen. Die FDA (Federal Drug Administration, amerikanische Gesundheitsbehörde) nennt als mögliche Komplikationen Hirnschäden, epileptische Anfälle, Bewusstlosigkeit und Herzrhythmusstörungen.

Als pharmazeutisch verträgliche Salze kommen erfindungsgemäß alle gängigen Salze in Frage wie Salze mit anorganischen Basen wie Halogenide (Chloride, Bromide, Iodide, Fluoride), Hydrohalogenide wie Hydrochlorid, Hydrobromid, Hydroiodid, Ammoniumsalze, Sulfide,, Hydrosulfide, Phosphate, Diphosphate, Sulfate, Carbonate, Hydrogencarbonate, Nitrate, Salze mit organischen Basen wie z.B. das Acetat, Maleat, Fumarat, Tartrat, Mesylat oder Toluolsulfonat, oder mit basischen Aminosäuren. Besonders bevorzugt ist das Hydrobromid, das auch in den üblichen kommerziell erhältlichen Präparaten verwendet wird.

Erfindungsgemäß sind auch Hydrate oder Solvate von dem Begriff "Dextromethorphan oder pharmazeutisch verträgliche Salze davon" umfasst. Am bevorzugtesten wird erfindungsgemäß Dextromethorphan-HydrochloridMonohydrat verwendet.

Es gibt auf dem Markt einige Kombinationspräparate, die Dextromethorphan enthalten, wie z.B. Wick Medinait®, das als Wirkstoffe Paracetamol, Dextromethorphanhydrobromid, Ephedrinhemisulfat und Doxylaminsuccinat enthält, und Wick DayMed®, enthaltend Paracetamol, Dextromethorphanhydrobromid, Phenylpropanolaminhydrochlorid.

Auch Kombinationen von Dextromethorphan mit Erkältungs-Hausmitteln wie Honig sind kommerziell erhältlich, z.B. Wick® Hustenpastillen gegen Reizhusten mit Honig.

Liquor Ammonii anisatus ist ein gemäß dem Deutschen Arzneicodex (DAC) hergestellter standardisierter ammoniakalischer Anisextrakt. Er enthält Ethanol, Liquor Ammonii anisatus wird auch als "anisölhaltige Ammoniak-Lösung" oder "zusammengesetzter Anisspiritus" bezeichnet. Weitere Synonyme sind Ammoniaklösung, anisölhaltige (DAC) , Anisammoniak, Ammonii hydroxidi solutio anisata, Spiritus ammonii anisatus" Anisierter Ammoniakgeist (PH), Anisölhaltige Ammoniak-Lösung (ASK), Ammoniaque anisee, Liquor di ammonio anisato, Anisölhaltige Ammoniakflüssigkeit, Zusammengesetzter Anisspiritus, Spiritus Anisi compositus, Anisspiritus, zusammengesetzter (ÖAB). Alle diese Synonyme sind vom Unfang der vorliegenden Erfindung umfasst und zur erfindungsgemäßen Verwendung geeignet. Liquor Ammonii anisatus wird aus Anisöl hergestellt, das wiederum nach DAB 9 (Deutsches Arzneibuch 9) ein aus den reifen trockenen Früchten von Anis (Pimpinella anisum) bzw. Sternanis (Illicium verum) durch Wasserdampfdestillation erhaltenes ätherisches Öl ist.

Die Herstellung und Prüfung von Liquor Ammonii anisatus erfolgt nach dem DAC 2003, A-1 00, wie folgt: 3,3 g Anisöl werden in 80,0 g Ethanol (90 Vol.-%) gelöst und anschließend mit 16,7 g 10 %-iger (Vol.-%) Ammoniaklösung versetzt. Man erhält eine klare, farblose bis schwach gelbe Flüssigkeit, die stark nach Anis und Ammoniak riecht.

Anisöl ist als Hausmittel gegen zahlreiche Beschwerden seit langem in Gebrauch, es wird ihm z.B. eine schleimlösende, auswurffördernde, krampflösende, blähungswidrige, harntreibende, milchbildende, verdauungsfördernde und appetitanregende sowie antibakterielle Wirkung zugeschrieben. In der Volksmedizin wurde es wegen seiner schleimlösenden Wirkung als Hustenmittel verwendet. Anistee, der durch Überbrühen von Anisfrüchten mit kochendem Wasser gewonnen wird, soll gegen Krämpfe und Blähungen helfen.

Das erfindungsgemäße Präparat wird z.B. hergestellt durch Mischen von Dextromethorphan oder einem pharmazeutisch verträglichen Salz davon, wie dem Hydrobromod-Monosolvat, mit Liquor Ammonii anisatus, bis eine homogene Lösung erhalten wird. Zum Lösen des Dextromethorphan ist der im Liquor Ammonii anisatus vorhandene Alkohol notwendig. Unter Alkohol wird erfindungsgemäß Ethanol verstanden, Anschließend kann der Alkohol auf übliche Weise entfernt werden, z.B. durch Abdampfen.

Im erfindungsgemäßen Präparat werden bevorzugt Dextromethorphan oder ein pharmazeutisch verträgliches Salz und Liquor Ammonii anisatus in Mengen von 0,005 bis 0, 1 g, bevorzugt 0,01 bis 0,04 g, besonders bevorzugt 0,02 g Dextromethorphanhydrobromid-Monohydrat und 0,5 bis 3,0, bevorzugt 1,0 bis 2,5, besonders bevorzugt 2,0 g Liquor Ammonii anisatus kombiniert. Das Gewichtsverhältnis der beiden Wirkstoffe beträgt dabei bevorzugt Dextromethorphan-Hydrobromid-Monohydrat : Liquor Ammonii anisatus 1:80 bis 1:1 20, bevorzugt 1:90 bis 1:10, besonders bevorzugt 1:100. In einer Ausführungsform wird die Kombination mit Wasser gemischt, um eine anwendungsfreundliche Konzentration zu erzielen. Dabei wird bevorzugt Aqua purificata (gereinigtes Wasser) eingesetzt. Die Menge an Wasser kann beliebig gewählt werden, wird aber bevorzugt so eingestellt, dass die Gesamtwirkstoffkonzentration (Summe der Gewichtsanteile von Dextromethorphan-Hydrobromid-Monohydrat und Liquor Ammonii anisotus) 5 bis 50 Gew.-%, bevorzugt 10 bis 25 Gew.-% und am bevorzugtesten 20-21 Gew.-% beträgt. Das Präparat hat daher besonders bevorzugt folgende Zusammensetzung:

| | |
|---|---|
| Dextromethorphan-Hydrobromid-Monohydrat | 0,02 g |
| Liquor Ammonii anisatus | 2,00 g |
| Aqua purificata | ad 10,00 g |

Es wurde gefunden, dass mit dieser Zusammensetzung eine besonders gute hustenreizstillende Wirkung bei gleichzeitig geringer Verschleimung auftritt.

Als Expectorantien geeignet sind erfindungsgemäß weiterhin Fenchelöl, Efeuextrakt, Thymianöl, Ambroxol, Acetylcystein, Carbocystein, Bromhexin und Guiafenesin, die erfindungsgemäß in alkoholischer Lösung eingesetzt werden.

Erfindungsgemäß kann das Präparat bevorzugt als Hustentropfen vorliegen, die entweder pur, mit Wasser, Saft oder Tee verdünnt oder auch auf ein Zuckerstückchen aufgetropft eingenommen werden können.

Es ist auch möglich, die erfindungsgemäße Wirkstoffkombination zur Herstellung von Hustensaft in eine geeignete Sirupgrundlage einzumischen, z.B. Saccharose, Xylitol, D-Xylose, Maltose, D-Glucose, Sorbitol, Glycerin, Mannitol, Sirupus simplex oder ähnliche. In diesem Fall wird dann kein Wasser zur Verdünnung eingesetzt. Das erhaltene Präparat kann dann unverdünnt als Hustensaft eingenommen werden.

Die erfindungsgemäße Wirkstoffkombination kann auch als Füllung von Hustenbonbons oder in Lutschpastillen konfektioniert werden.

Die erfindungsgemäße Wirkstoffkombination kann übliche pharmazeutische Hilfsstoffe wie Exzipienten, Verdicker, Verdünner, Emulgatoren Konservierungsstoffe, Antioxidantien, Geschmacks- und Aromastoffe, Farbstoffe, Puffer, Stabilisatoren oder ähnliche enthalten.

Die erfindungsgemäße oben angegebene bevorzugten Hustentropfen werden in Abhängigkeit vom Alter des Patienten und der Schwere der Erkrankung in einer Dosis von fallweise 15-20 Tr. bei Kindern, 30-40 Tropfen bei Erwachsenen eingenommen. Üblicherweise ist das tägliche Einnahmeschema abhängig vom Grad der Beschwerden und liegt bei 2 bis 5 mal täglich, bevorzugt 2 bis 3 mal täglich.

Durch die erfindungsgemäße Wirkstoffkombination wird eine überraschend gute Besserung des Reizhustens erzielt, die mit anderen üblichen Hustenmitteln nicht erreicht wird. Besonders bei Asthmatikern wird eine besonders gute Linderung des Reizhustens beobachtet, ohne dass eine verstärkte Schleimbildung auftritt, die sich bei dieser Grundkrankheit besonders nachteilig auswirken würde. Dies ist besonders überraschend, da allgemein eine Kombination von Antitussiva mit Expectorantien als nachteilig und kontraproduktiv angesehen wird.

### Beispiel 1:

### Hustentropfen

20,0 mg Dextromethorphan-Hydrobromid-Monohydrat, 2,00 g Liquor Ammonii anisatus (Fertigprodukt nach DAC 2003) und Wasser ad 10,00 g wurden bei Raumtemperatur gemischt, bis keine festen Bestandteile mehr erkennbar waren, und in getönte Glastropfflaschen abgefüllt.

### Beispiel 2:

### Anwendung bei einem Asthmapatienten mit Reizhusten

Der Patient, der seit Jahren an Asthma leidet und deswegen mit Terbutalin und Cortisonpräparaten medikamentiert wird, klagte über Reizhusten im Rahmen einer Erkältung. Er nahm die Hustentropfen gemäß Beispiel 1 während 7 Tagen 3 mal täglich in Wasser verdünnt ein. Schon nach wenigen Stunden berichtete er über eine deutliche Besserung des Reizhustens, die nicht von einer verstärkten Schleimbildung in den Bronchien begleitet war. Diese Besserung wurde als wesentlich besser als mit anderen Antitussiva beschrieben.

## Patentansprüche

1. Pharmazeutisches Präparat, welches als Wirkstoffe Dextromethorphan oder ein pharmazeutisch verträgliches Salz davon und Liquor Ammonii anisatus umfasst.

2. Präparat nach Anspruch 1, wobei als Salz von Dextromethorphan das Dextromethorphan-Hydrobromid-Monohydrat verwendet wird.

3. Präparat nach Anspruch 2, wobei die Bestandteile in folgenden Gewichtsverhältnissen vorliegen:
Dextromethorphan-Hydrobromid-Monohydrat : Liquor Ammonii anisatus = 1 :80 bis 1:120, bevorzugt 1 :90 bis 1:110, besonders bevorzugt 1:100.

4. Präparat nach einem der Ansprüche 1 bis 3, darüber hinaus umfassend Wasser.

5. Präparat nach einem der Ansprüche 1 bis 4 zur Verwendung in einem Verfahren zur Behandlung von Husten.

6. Präparat nach einem der Ausprüche 1 bis 4 zur Verwendung in einem Verfahren zur Behandlung von Reizhusten bei Asthmatikern.

7. Präparat zur Verwendung in einem Verfahren nach einem der Ansprüche 5 oder 6 in Form von Hustentropfen in wämiger Form.

## Claims

1. Pharmaceutical preparation comprising as active ingredients dextrometorphan or a pharmaceutically acceptable salt thereof and liquor ammonii anisatus.

2. Preparation according to claim 1, wherein dextrometorphan-hydrobromide-monohydrate is used as salt of dextrometorphan.

3. Preparation according to claim 2, wherein the components are present in the following ratios:
Dextrometorphan-hydrobromide-monohydrate : liquor ammonii anisatus = 1:80 to 1:120, preferably 1 :90 to 1:110, more preferably 1:100.

4. Preparation according to one of claims 1 to 3, further comprising water.

5. Preparation according to one of claims 1 to 4 for use in a method for treating cough.

6. Preparation according to one of claims 1 to 4 for use in a method for treating reflex cough of asthmatics.

7. Preparation for use in a method according to one of claims 5 or 6 or in the form of cough drops in aqueous form.

## Revendications

1. Composition pharmaceutique comprenant en tant qu'agent actif du dextromethorphane ou un sel pharmaceutiquement acceptable de celui-ci et le liquor Ammonii anisatus.

2. Composition selon la revendication 1, dans laquelle on utilise en tant que sel de dextromethorphane le dextromethorphane hydrobromide monohydrate.

3. Composition selon la revendication 2, dans laquelle les constituants sont présents dans les rapports pondéraux suivants:
dextromethorphane hydrobromide monohydrate:Liquor Ammonii anisatus = 1:80 à 1:120, préférentiellement 1:90 à 1:110, encore plus préférentiellement 1:100.

4. Composition selon l'une des revendications 1 à 3, comprenant en outre de l'eau.

5. Composition selon l'une des revendications 1 à 4, pour l'utilisation dans un procédé pour le traitement de la toux.

6. Composition, pour l'utilisation dans un procédé selon l'une des revendications 1 à 4 pour le traitement de la toux irritante chez les asthmatiques.

7. Composition pour l'utilisation dans un procédé selon l'une des revendications 5 ou 6 dans la forme de gouttes contre la toux en forme aqueuse.
